# DEMANDE DE BREVET EUROPEEN

(11) **EP 1 987 833 A1**
(43) Date de publication de la demande: **05.11.2008**
(21) Numéro de dépôt: 08161558.5
(22) Date de dépôt: 30.12.2005
(51) Int. Cl.: A61K 31/4468, A61K 31/395, A61P 43/00

(54) **Utilisation de benzoxazines pour la fabrication d'un médicament destiné à la prévention ou au traitement de la tolérance aigue aux morphiniques**

(30) Priorité: 06.01.2005 FR 0500112
(62) Demande divisionnaire de: 05292822.3
(71) Demandeur: Biocodex, 94250 Gentilly (FR); UNIVERSITE DE POITIERS, 86034 Poitiers Cedex (FR)
(72) Inventeur: Tirault, Myriam, 86000 Poitiers (FR); Debaene, Bertrand, 86000 Poitiers (FR); Le Guern, Marie-Emmanuelle, 60200 Compiegne (FR); Hublot, Bernard, 60200 Compiegne (FR)
(74) Mandataire: Jacobson, Claude

(57) **Abrégé**

La présente invention concerne un composé de formule générale (I) suivante : 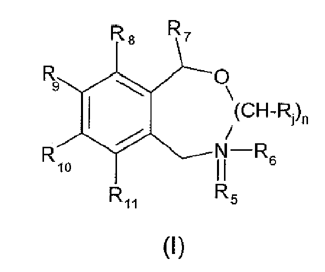 ou un sel pharmaceutiquement acceptable de celui-ci,
pour son utilisation dans la prévention ou le traitement de la tolérance aiguë aux morphiniques, chez un individu ayant reçu une administration d'un analgésique morphinique.

## Description

La présente invention concerne, en particulier, l'utilisation de composés pharmacologiques afin de prévenir ou traiter la tolérance aiguë aux morphiniques.

La morphine et ses dérivés synthétiques (fentanyl, sufentanil, alfentanil, rémifentanil) sont de puissantes molécules antalgiques appelées opioïdes ou morphiniques, utilisées au cours de l'anesthésie générale, pour traiter les douleurs liées à la chirurgie. Il existe une grande variabilité interindividuelle des besoins en morphiniques pour une même opération.

Les données scientifiques récentes laissent penser que plus la dose de morphinique administrée pendant l'intervention est grande, plus les besoins en antalgiques sont importants au cours de la période post-opératoire immédiate, notamment pour traiter les effets hyperalgésiques induits par ces morphiniques. Ce phénomène porte le nom de tolérance aiguë aux morphiniques.

La tolérance aiguë aux morphiniques se définit par l'augmentation de la consommation post-opératoire de morphine, du fait de l'exposition ponctuelle à des opioïdes au cours de l'anesthésie. Elle semble apparaître d'autant plus rapidement que le morphinique est puissant, de courte durée d'action, et que de fortes doses sont utilisées (Guignard et al. (2000) Anesthesiology 93 : 409-17).

La tolérance aiguë post-opératoire se distingue en particulier de la tolérance chronique, qui est l'épuisement de l'efficacité antalgique du morphinique au long cours, mise en évidence chez les patients cancéreux douloureux chroniques, avec corrélativement, la nécessité d'augmenter les doses pour obtenir le même effet (Muller et al. (1999) Ann Fr Anesth Réanim 18 : 866-95).

Le phénomène de tolérance aiguë aux morphiniques a déjà été montré chez l'animal. Ainsi, l'injection répétée de fentanyl chez le rat (4 doses en 1h) conduit, après stimulation nociceptive intense (pressions d'intensités croissantes sur la patte arrière), à deux types de réponse:
- effet précoce d'analgésie, avec augmentation du seuil douloureux (c'est-à-dire possibilité d'appliquer une pression d'intensité supérieure, sans entraîner de réponse motrice du rat),
- effet retardé d'hyperalgésie, avec diminution du seuil douloureux, d'autant plus profonde que la dose totale de fentanyl administrée est grande.

D'après l'auteur, cette sensibilisation à la douleur est due à l'opioïde lui-même, et non à un conditionnement induit par le stimulus nociceptif périphérique répété en excès ; en effet, l'augmentation de la douleur du rat, après injection d'une forte dose de fentanyl, apparaît même lorsque le rat n'a pas subi d'agression nociceptive intense (Celerier et al. (2000) Anesthesiology 92 : 465-72).

Chez l'homme, les résultats sont comparables. En effet, on observe chez le volontaire sain, après 90 minutes d'une perfusion continue de rémifentanil, une décroissance marquée de la durée pendant laquelle le sujet peut supporter l'application d'un stimulus thermique (eau froide) ou d'un stimulus mécanique (pression sur la main non dominante) (Vinik et al. (1998) Anesth Analg 86 : 1307-11).

L'existence de ce phénomène de tolérance aiguë aux morphiniques a également été rapporté en situation clinique :
- après hystérectomie par laparotomie; dans ce cas, chez les patientes ayant reçu pendant l'anesthésie de fortes doses de fentanyl (15 µg /kg), la quantité de morphinique nécessaire au contrôle des douleurs post-opératoires est accrue, augmentant ainsi le risque de survenue d'effets indésirables (dépression respiratoire, nausées et vomissements notamment) (Chia et al. (1999) Can J Anesth 46 : 872-7).
- après laparotomie pour chirurgie colo-rectale d'au moins deux heures ; dans ce cas le rémifëntanil à fortes doses entraîne un effet de tolérance aiguë, qui se manifeste cliniquement par une consommation plus importante de morphine en salle de réveil (Guignard et al. (2000) Anesthesiology 93 : 409-17).

Ainsi, l'utilisation de fortes doses per-opératoires de morphiniques contribue à une augmentation des besoins antalgiques post-opératoires (Eisenach (2000) Anesthesiology 92 : 308-9).

Toutefois, il n'est pas envisageable à l'heure actuelle de supprimer les morphiniques au cours de l'anesthésie, car ils sont indispensables, mais plutôt de trouver la molécule qui, lorsqu'elle leur est associée, empêche l'émergence du phénomène.

Il a été montré que les agents volatils halogénés (utilisés en entretien de l'hypnose dans toutes les études précédentes), qui ont une activité anti-NMDA, atténuent en partie la tolérance aiguë aux morphiniques (Chauvin (2001) La Collection de la SFAR (Elsevier) ; Evaluation et traitement de la douleur 99-108).

De même, la kétamine, antagoniste des récepteurs NMDA, réduit, mais sans l'annuler, la tolérance aiguë à l'alfentanil chez le rat (Kissin et al. (2000) Anesth Analg 91 : 1483-8). Elle agit par réduction de l'hyperexcitabilité des cellules de la corne postérieure de la moelle et de la transmission glutamatergique, induites par le fentanyl (Celerier et al. (2000) Anesthesiology 92 : 465-72).

L'efficacité de ces produits est limitée, de plus ils présentent des effets indésirables. Par conséquent, la présente invention a pour objet de fournir des composés ayant une efficacité au moins comparable à ceux déjà connus et dépourvus de leurs effets indésirables pour traiter la tolérance aiguë aux morphiniques.

La présente invention découle de la mise en évidence par les Inventeurs que, de manière inattendue, le néfopam, une benzoxazine, permettait de prévenir la tolérance aiguë aux morphiniques (notamment lorsque le néfopam est administré avant le réveil du patient ayant subi une intervention chirurgicale durant laquelle il a été anesthésié avec des doses d'analgésiques morphiniques usuelles pour une anesthésie générale).

Ainsi, la présente invention concerne l'utilisation d'au moins un composé de formule générale (I) suivante : 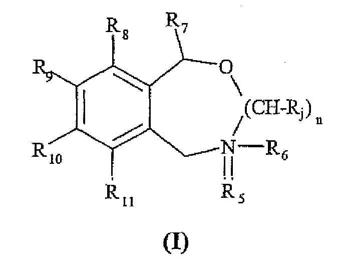 dans laquelle :
- R₅ représente O ou aucun groupement ;
- R₆ représente H ou un groupement alkyle contenant de 1 à 6 atomes de carbone ;
- n représente un nombre entier de 2 à 4 ;
- j représente un nombre entier variant de 1 à n ;
- Rⱼ, identique ou différent pour chaque carbone substitué, représente H ou un groupement alkyle contenant de 1 à 6 atomes de carbone ;
- R₇ représente un groupement phényle éventuellement substitué par un ou plusieurs groupements, identiques ou différents, choisis parmi la liste comprenant H, un groupement alkyle contenant de 1 à 6 atomes de carbone, un groupement alkoxy contenant de 1 à 6 atomes de carbone, un groupement trifluorométhyle, ou un atome d'halogène ;
- R₈, R₉, R₁₀, R₁₁ identiques ou différents représentent H, un groupement alkyle contenant de 1 à 6 atomes de carbone, un groupement alkoxy contenant de 1 à 6 atomes de carbone, un groupement trifluorométhyle, ou un atome d'halogène ;
ou de ses sels pharmaceutiquement acceptables,
pour la préparation d'un médicament destiné à la prévention ou au traitement de la tolérance aiguë aux analgésiques morphiniques ou de l'hyperalgésie due aux analgésiques morphiniques, chez un individu ayant reçu une administration d'un analgésique morphinique, notamment à une dose suffisante dans le cadre d'une intervention chirurgicale.

L'invention concerne plus particulièrement l'utilisation telle que définie ci-dessus des composés de formules (Ia) et (Ib) suivants ou de leurs mélanges :

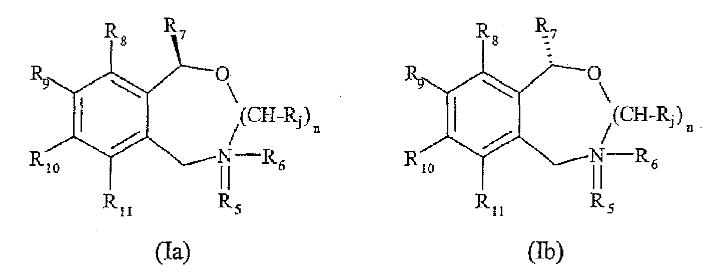

Des composés de formule (I) ainsi que leur synthèse sont notamment décrits dans le fascicule de brevet FR 1 590 067. Les adaptations nécessaires à la synthèse de l'ensemble des composés de formule (I) sont aisées pour l'homme du métier.

On désigne par « analgésique morphinique » la morphine ou un dérivé de la morphine. En particulier les analgésiques morphiniques répondent à la formule suivante : 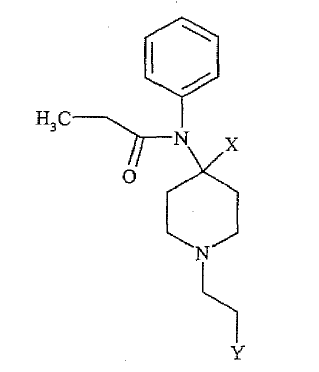 dans laquelle :
- X représente H, un groupe alkoxyalkyle de 2 à 5 atomes de carbone, ou un groupe alkoxycarbonyle de 2 à 5 atomes de carbone ;
- Y représente un groupe aryle ou hétéroaryle de 5 à 8 atomes éventuellement substitué par un ou plusieurs groupes alkyles de 1 à 5 atomes de carbone, ou un groupe akoxycarbonyles de 2 à 5 atomes de carbone ;

On désigne par « tolérance aiguë aux morphiniques » le phénomène pharmacologique par lequel la dose de morphine nécessaire au soulagement de douleurs post-opératoires, notamment après une intervention chirurgicale sous anesthésie, est accrue, du fait de l'exposition préalable et ponctuelle, au cours de l'anesthésie, à des analgésiques morphiniques.

On désigne par « hyperalgésie » la perception anormalement intense d'un stimulus douloureux.

On désigne par « hyperalgésie due aux morphiniques ? » le fait que l'exposition à des morphiniques induit une sensibilisation à la douleur, qui se manifeste cliniquement par une augmentation des scores de douleur pour un même stimulus douloureux.

Les scores de douleur peuvent être notamment mesurés à l'aide de l'Echelle Visuelle Analogique (E.V.A.) bien connue de l'homme du métier.

Avantageusement, selon l'invention, les composés de formule (I) présentent la propriété de s'opposer aux phénomènes de la tolérance aiguë aux morphiniques ou de l'hyperalgésie due aux morphiniques. Cette propriété est distincte de leurs propriétés analgésiques.

Dans un mode de réalisation, l'invention concerne particulièrement l'utilisation telle que définie ci-dessus, de composés de formule générale (I), correspondant :
- au néfopam de formule (II) suivante, 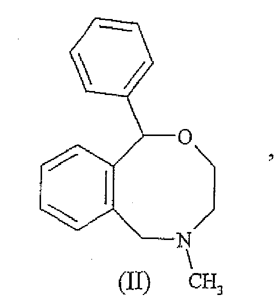 ou
- au desméthyle néfopam de formule (III) suivante, 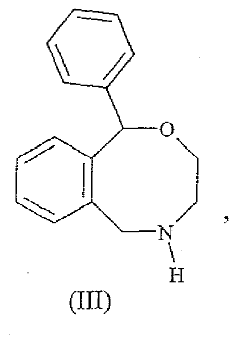 ou
- au *N*-oxyde néfopam de formule (IV) suivante,

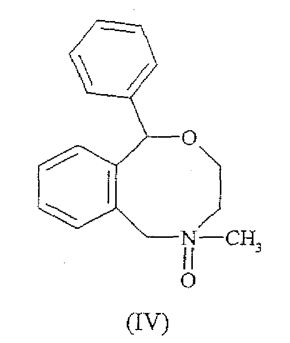

Il est entendu que l'invention concerne également l'utilisation telle que définie ci-dessus des composés (IIa), (IIb), (IIIa), (IIIb), (IVa) et (IVb) suivants ainsi que de leurs mélanges :

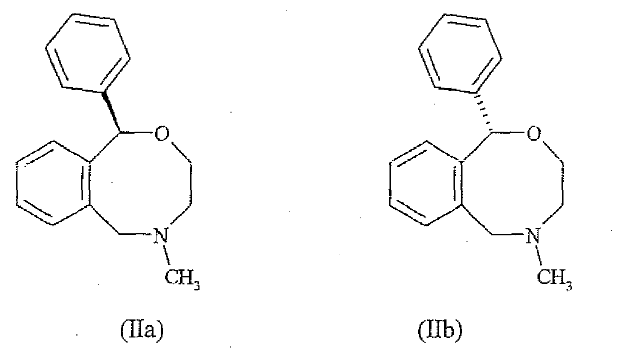

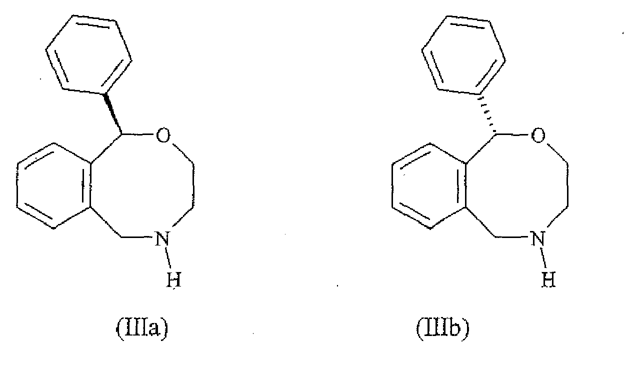

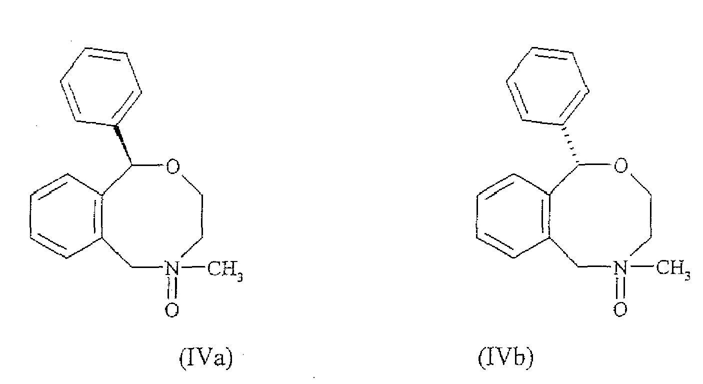

Dans un autre mode de réalisation, l'invention concerne plus particulièrement l'utilisation telle que définie ci-dessus, du néfopam de formule (II) suivante, 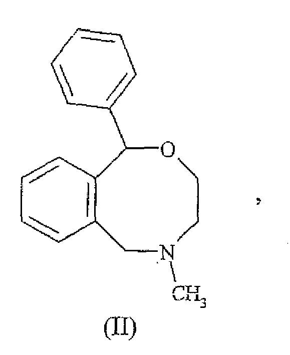 ou des sels pharmaceutiquement acceptables du néfopam,
pour la préparation d'un médicament destiné à la prévention ou au traitement de la tolérance aiguë aux morphiniques ou de l'hyperalgésie due aux morphiniques, chez un individu ayant reçu une administration d'un analgésique morphinique.

Le néfopam est le principe actif de l'Acupan®. Il est principalement utilisé en tant qu'analgésique lors d'interventions chirurgicales. Par ailleurs, ce composé présente des effets d'épargne de la morphine. Ces effets sont distincts des effets empêchant la survenue de la tolérance aiguë aux morphiniques ou de l'hyperalgésie due aux morphiniques qui ont été mis en évidence par les Inventeurs.

Un sel pharmaceutiquement acceptable préféré du néfopam est le chlorhydrate de néfopam.

Dans un autre mode de réalisation particulier de l'utilisation définie ci-dessus, le médicament convient pour l'administration d'une dose unitaire d'environ 1 mg à environ 120 mg, notamment d'environ 20 mg de chlorhydrate de néfopam, pour une posologie d'environ 1 mg/j à environ 120 mg/j, notamment d'environ 20 mg/j.

Selon un autre mode de réalisation préféré de l'utilisation définie ci-dessus, le médicament convient pour une administration par voie intraveineuse, par voie intramusculaire, ou par voie orale.
Selon un autre mode de réalisation particulièrement préféré de l'utilisation définie ci-dessus, le morphinique analgésique a été administré au cours d'une intervention chirurgicale (à savoir en phase per-opératoire), et plus particulièrement à une dose usuelle suffisante dans le cadre d'une intervention chirurgicale, notamment pour une anesthésie générale.

Selon un autre mode de réalisation particulièrement préféré de l'utilisation définie ci-dessus, le morphinique analgésique est choisi parmi le groupe comprenant le fentanyl, le rémifentanil, le sufentanil, ou l'alfentanil.

Les formules de ces composés sont présentées ci-dessous :

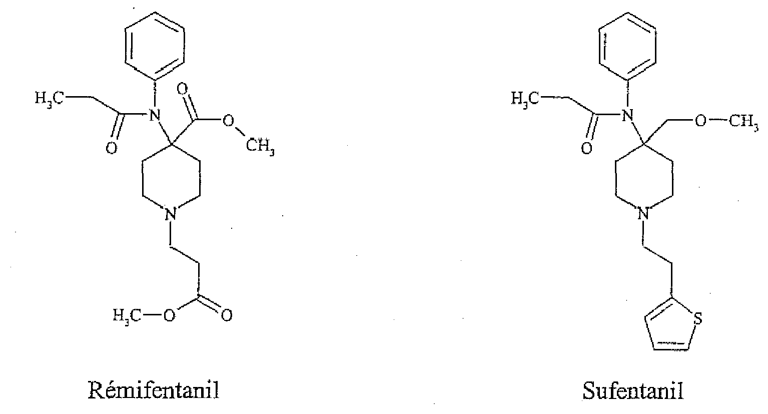

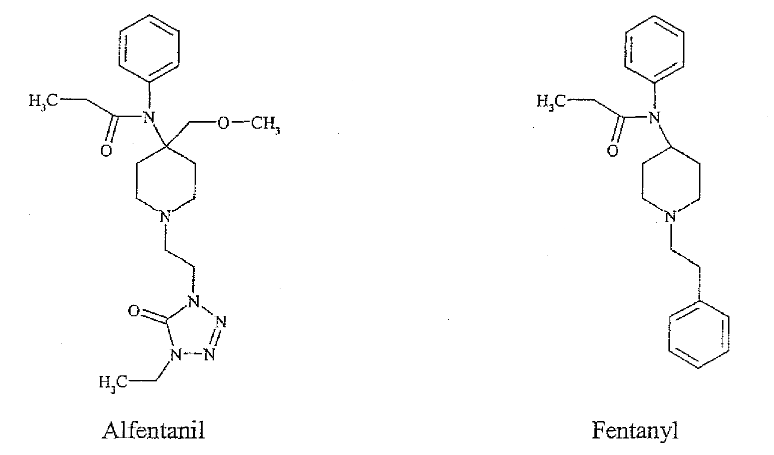

La présente invention concerne également des produits contenant :
- au moins un composé de formule (I) tel que défini ci-dessus, et
- au moins un analgésique morphinique,
comme produit de combinaison pour une utilisation simultanée, séparée ou étalée dans le temps pour induire une analgésie chez un patient et pour prévenir ou traiter la tolérance aiguë aux morphmiques ou l'hyperalgésie due aux morphiniques, liée à l'utilisation dudit analgésique morphinique chez ledit patient.

Dans un mode de réalisation particulier des produits définis ci-dessus, le composé de formule (I) est le néfopam, le desméthyle néfopam, ou le *N*-oxyde néfopam, notamment le néfopam.

Dans un mode de réalisation encore plus particulier des produits définis ci-dessus, l'analgésique morphinique est choisi parmi le groupe comprenant le fentanyl, le rémifentanil, le sufentanil, ou l'alfentanil.

Dans un mode de réalisation préféré des produits définis ci-dessus, l'analgésique morphinique est administré au patient pour produire un effet analgésique au cours d'une intervention chirurgicale, et le composé de formule (I), notamment le néfopam, est également administré au patient durant l'intervention chirurgicale, en particulier avant le réveil dudit patient (à savoir en fin de phase per-opératoire, et avant la phase post-opératoire).

En particulier, le néfopam est administré environ 30 minutes avant le réveil du patient.

### DESCRIPTION DES FIGURES

### Figure 1A, Figure 1B et Figure 1C

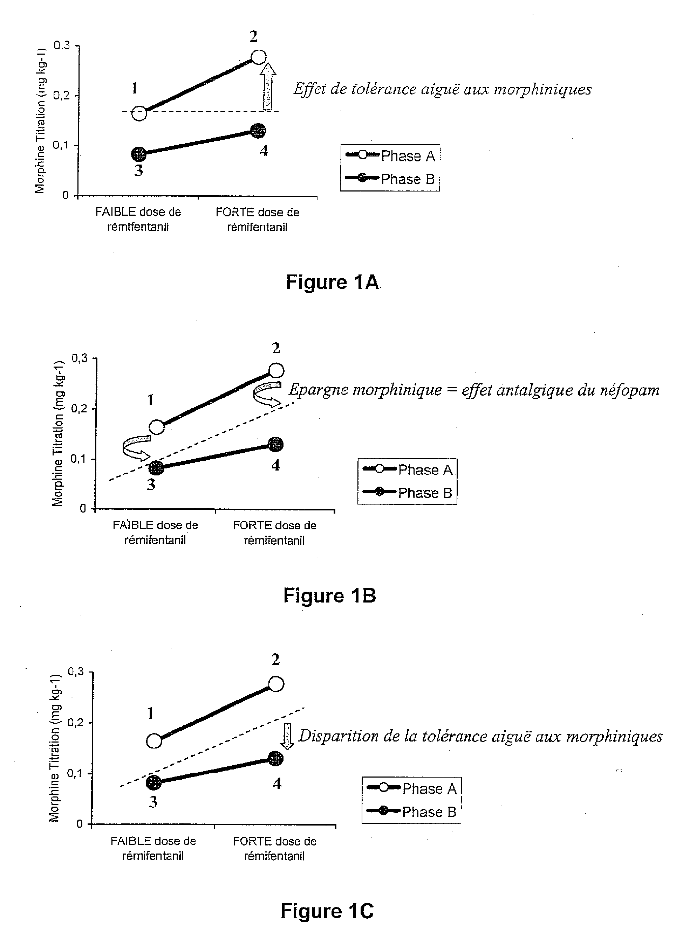

Les Figures 1A, 1B et 1C représente la quantité de morphine administrée en titration (axe des ordonnées, en mg/kg) à des groupes de patients ayant reçu une faible dose (1 et 3) ou une forte dose (2 et 4) de rémifentanyl (axe des ordonnées, unités arbitraires) en présence (3 et 4, phase A, cercles noirs) ou en absence (1 et 2, phase B, cercles blancs) de néfopam

La Figure 1A illustre l'effet de tolérance aiguë aux morphiniques ; c'est-à-dire l'augmentation de la quantité de morphine administrée en titration (flèche) induite par l'utilisation d'une forte dose de rémifetanyl (2) par rapport à la quantité de morphine administrée en titration pour une faible dose de rémifentanyl (1, droite en pointillés). En présence de néfopam (3 et 4) la variation de la quantité de morphine administrée en titration est non significative.

La Figure 1B illustre l'effet analgésique propre du néfopam ; la droite en pointillé indique quelle aurait dû être la quantité de morphine administrée en titration pour le groupe 4 (flèche supérieure) si le néfopam n'avait que des effets analgésiques.

La Figure 1C illustre l'inhibition de l'effet de tolérance aiguë aux morphiniques par le néfopam ; c'est-à-dire la diminution de la quantité de morphine administrée en titration pour le groupe 4 (flèche) par rapport à la quantité de morphine qui aurait du être administrée si le néfopam n'avait eu que des effets analgésiques (droite en pointillés).

### EXEMPLE

L'étude réalisée par les Inventeurs a été divisée en deux phases :
- phase A : confirmation du phénomène de tolérance aiguë aux morphiniques,
- phase B : mise en évidence du rôle inhibiteur du néfopam sur ce phénomène de tolérance.

### 1. Constitution de la cohorte de l'étude

Soixante patients hospitalisés pour une laparotomie en chirurgie digestive ont été enrôlés dans l'étude. Après information et consentement éclairé, les patients ont été randomisés en 2 groupes pour la phase A (groupes 1 et 2), et 2 groupes pour la phase B (groupes 3 et 4).

Les patients des groupes 1 et 3 ont reçu une dose faible d'un analgésique morphinique, le rémifentanil, lors de l'anesthésie (cible cérébrale 3 ng/ml), alors que les patients des groupes 2 et 4 ont reçu une dose élevée de rémiféntanil (cible cérébrale 8 ng/ml). En outre, les patients des groupes 3 et 4 ont reçu du néfopam (20 mg) avant la fin de l'anesthésie.

Les caractéristiques de ces patients sont résumées dans le **Tableau 1** suivant :

**Tableau 1 : caractéristiques des patients inclus dans l'étude**

| | **Groupe 1** | **Groupe 2** | **Groupe 3** | **Groupe 4** |
|---|---|---|---|---|
| | **(n = 13)** | **(n = 15)** | **(n = 14)** | **(n = 15)** |
| Répartition par sexe (H/F) | 8/5 | 9/6 | 6/8 | 11/4 |
| Age (années) | 58 [39-68] | 55 [19-65] | 56 [43-67] | 63 [50-70] |
| Poids (kg) | 75 [48-91] | 66 [48-100] | 66 [45-108] | 77 [54-105] |
| Taille (cm) | 168 [151-178] | 172 [155-185] | 165 [151-174] | 171 [155-180] |
| IMC (cm/kg²) | 27 [20-34] | 25 [18-32] | 25 [17-35] | 27 [18-33] |
| ASA I, II, III | 2, 11, 0 | 3, 12, 0 | 3, 10, 1 | 1, 10, 4 |

| | | | | |
|---|---|---|---|---|
| IMC : indice de masse corporelle ASA : score de l'American Society of Anesthesiologists | | | | |

### 2. Période per-opératoire

1h à 2h avant le début de la chirurgie les patients ont reçu 1 mg/kg per os d'hydxoxyzine (Atarax®) (traitement habituel indispensable).

Puis les patients ont bénéficié d'un protocole d'anesthésie utilisant :
- comme myorelaxant ; l'atracurium
- comme hypnotique : le propofol
- comme analgésique morphinique : le rémifentanil

Afin de répondre de la façon la plus juste et rapide aux stimuli chirurgicaux, il a été choisi de délivrer l'hypnotique et le morphinique par voie intraveineuse, grâce à un pousse-seringue électronique piloté par un logiciel informatique. Ce procédé porte le nom d' A.I.V.O.C : Anesthésie Intraveineuse à Objectif de Concentration. L'ordinateur intègre les modèles pharmacocinétiques du propofol et du rémifentanil et calcule à chaque instant et selon les caractéristiques physiques du patient (âge, sexe, poids), la concentration-cible plasmatique ou cérébrale à atteindre pour un niveau d'anesthésie optimum, et adapte le débit de perfusion en conséquence.

Ce type de perfusion adaptée aux besoins réels du patient en produits anesthésiques nécessite un équipement particulier :
- Pousse-seringue *Diprifusor,* pour l'A.I.V.O.C de propofol (dilution 10 mg/ml) : il permet une adaptation rapide du degré d'hypnose.
- Pousse-seringue *Fresenius Vial,* commandé par le logiciel *Stanpump* sur ordinateur portable, pour l'A.I.V.O.C de rémifentanil (dilution 50 µg / ml).

Des études précédentes ont permis de déterminer les concentrations cibles à atteindre en fonction de la progression de l'anesthésie pour obtenir un niveau d'hypnose donné, pour le propofol, ou d'analgésie donnée, pour le rémifentanil.

| **ETAPE** | **PROPOFOL** | **REMIFENTANIL** |
|---|---|---|
| | concentration cible plasmatique - µg/ml | concentration cible plasmatique - ng/ml |
| Intubation | 4 à 8 | 4 (associé au propofol) |
| Entretien | 3 à 6 | 3 à 12 (associé au propofol) |
| Réveil | 1 à 2 | 1,6 (ventilation spontanée) |

### Induction de l'anesthésie :

- le propofol est administré en flash pour une cible plasmatique de 6 à 8 µg/ml,
- le rémifentanil est introduit 1 minute après le propofol pour une cible cérébrale de 4 ng/ml,
- l'atracurium est introduit ensuite par un bolus intraveineux (IV) de 0,5 mg/kg.

Le patient est alors intubé environ 4 minutes après l'administration de propofol.

### Entretien de l'anesthésie :

- pour tous les patients : maintien de la curarisation par bolus IV d'atracurium (injections itératives d'une demi-dose d'induction selon les données du curamètre) ;
- pour les patients de la phase A :
   - groupe 1 : cible cérébrale de rémifentanil fixée à 3 ng/ml,
   - groupe 2 : cible cérébrale de rémifentanil fixée à 8 ng/ml,
- pour les patients de la phase B :
   - groupe 3 : cible cérébrale de rémifentanil fixée à 3 ng/ml,
   - groupe 4 : cible cérébrale de rémifentanil fixée à 8 ng/ml,

La profondeur de l'anesthésie est ajustée en modulant l'administration de propofol selon la réponse hémodynamique à la chirurgie (cible plasmatique variable de 3 à 8 µg/ml).

### Fin de l'anesthésie :

Un relais antalgique per-opératoire est réalisé au début de la fermeture du péritoine en prévention des douleurs post-opératoires :
- pour les patients de la phase A (groupes 1 et 2) : morphine à 0,15 mg /kg en injection intraveineuse directe (IVD)
- pour les patients de la phase B (groupes 3 et 4) : morphine à 0,15 mg /kg en IVD + 20 mg de chlorhydrate de néfopam (Acupan®) en injection intraveineuse lente (IVL) sur 20 min.

A la fin des pansements, l'administration de rémifentanil et de propofol est interrompue et le patient est transféré en Salle de Surveillance Post-Interventionnelle (S.S.P.I.)

Les caractéristiques de l'anesthésie sont résumées dans le **Tableau 3** suivant :

**Tableau 3 : caractéristiques de l'anesthésie**

| | **Groupe 1** | **Groupe 2** | **Groupe 3** | **Groupe 4** |
|---|---|---|---|---|
| | **(n = 13)** | **(n = 15)** | **(n = 14)** | **(n = 15)** |
| Rémifentanil | 0,083 | 0,239 | 0,085 | 0,203 |
| (µg/kg/min) | [0,056-0,116] | [0,184-0,455] | [0,068-0,154] | [0,124-0.379] |
| Propofol | 0,199 | 0,138 | 0,186 | 0,131 |
| (mg/kg/min) | [0,119 - 0,318] | [0,110 - 0,194] | [0,132-0,265] | [0,011-0,221] |
| Bolus de morphine | 11 | 10 | 10 | 11 |
| (mg) | | | | |
| Durée d'Anesthésie | 204 | 233 | 198 | 181 |
| (min) | [160 - 245] | [90 - 367] | [90 - 325] | [130-357] |
| Durée de Chirurgie | 148 | 190 | 143 | 155 |
| (min) | [105-200] | [50-325] | [21-272] | [95-317] |
| Délai d'analgésie | 33 | 36 | 27 | 27 |
| préventive (min) | [13-42] | [20-80] | [13-58] | [20-69] |
| Délai d'Extubation | 34 | 25 | 39 | 24 |
| (min) | [10-105] | [12-65] | [9-74] | [12-108] |
| Délai de Titration | 37 | 10 | 17 | 20 |
| (min) | [5 - 90] | [1 - 63] | [1 - 57] | [6 - 70] |

| | | | | |
|---|---|---|---|---|
| durée de l'anesthésie: de l'heure d'induction à l'arrêt des produits d'anesthésie ; durée de la chirurgie : de l'heure d'incision à la fin des pansements ; délai de l'analgésie préventive : de l'injection per-opératoire du bolus préventif de morphine à l'arrêt des produits d'anesthésie ; délai d'extubation : de l'arrêt de l'anesthésie au retrait de la sonde d'intubation ; délai de titration : de l'extubation à la première injection de morphine en titration. | | | | |

### 3. Période post-opératoire :

A son réveil, il est demandé au patient de chiffrer sa douleur sur l'échelle E.V.A (Echelle Visuelle Analogique, graduée de 0 à 100). Il reçoit alors des injections intraveineuses répétées de morphine (bolus de 3 mg IVD), jusqu'à soulagement de sa douleur ( E.V.A ≤ 30). Cette procédure habituelle de traitement de la douleur aiguë post-opératoire s'appelle la TITRATION en morphine. Elle est réalisée en Salle de Surveillance Post-interventionnelle (S.S.P.I).

Par la suite, le patient est équipé d'une pompe à morphine sur une voie veineuse périphérique réservée, la P.C.A (Analgésie Contrôlée par le Patient) afin d'assurer l'analgésie dans le service d'hospitalisation. Elle est déclenchée par le patient lui-même lorsqu'il perçoit de nouveau une douleur : la pompe P.C.A (pompe MASTER-PCA de VIAL) délivre alors automatiquement un bolus d'antalgique (1 mg), dans les limites de consommation programmées par le médecin. La P.C.A permet l'auto-gestion de la douleur par le patient, en évitant le sous-dosage comme le surdosage en morphine. Il est largement admis que ce système réduit la survenue d'effets indésirables liés à l'utilisation de la morphine. La PCA-morphine est actuellement la référence dans le traitement des douleurs aiguës post-opératoires, et est largement diffusée.

La surveillance post-opératoire (analgésie, survenue d'effets indésirables) est effectuée en Salle de Réveil (S.S.P.I), puis dans le service d'hospitalisation.

Les résultats de l'étude sont présentés dans le **Tableau 4** ci-dessous :

**Tableau 4 : évaluation de la douleur post-opératoire**

| | **Groupe 1** | **Groupe 2** | **Groupe 3** | **Groupe 4** |
|---|---|---|---|---|
| | **(n=13)** | **(n=15)** | **(n=14)** | **(n = 15)** |
| Titration morphine | 0,164 | 0,277 | 0,082 | 0,130 |
| (mg/kg) | [0,033 - 0,407] | [0,039-0,375] | [0 - 0,319] | [0 - 0,350] |
| Titration morphine | 12 | 18 | 7,5 | 9 |
| (mg) | [3 - 24] | [3-27] | [0 - 15] | [0 - 24] |
| Patients non titrés | 0 | 0 | 4 | 1 |
| PCA-morphine sur 24h | 0,450 | 0,385 | 0,255 | 0,454 |
| (mg/kg) | [0,180- 0,770] | [0,050-1,010] | [0 - 1,492] | [0,095-0,871] |
| Total morphine sur 24h | 0,573 | 0,661 | 0,365 | 0,649 |
| (mg/kg) | [0,310 - 0,984] | [0,150 - 1,340] | [0 - 1,736] | [0,095 - 1,210] |

### 4. Analyse des résultats

Le critère de jugement principal est la dose de morphine en titration. Les résultats de l'étude ont été soumis à un test de Mann-Whitney et un évènement a été considéré significatif si p ≤ 0,05 avec un risque a de 5% et une puissance de 80%.

La comparaison des groupes 1 et 2 indique que l'utilisation de rémifentanil à forte dose (8 ng/ml) durant l'anesthésie induit une surconsommation statistiquement significative de morphine en titration de l'ordre de 68% par rapport à une utilisation de rémifentanil à faible dose (3 ng/ml).

Ceci confirme donc bien le phénomène de tolérance aiguë aux analgésiques morphiniques **(****Figure 1A****).**

Par ailleurs, la comparaison des groupes 3 et 4 indique que l'administration per-opératoire de 20 mg de chlorhydrate de néfopam abaisse la surconsommation de morphine en titration du groupe ayant reçu une forte dose de rémifentanil par rapport au groupe en ayant reçu une faible dose jusqu'à la rendre statistiquement non-significative **(****Figure 1A****).**

Ceci implique donc que l'administration de néfopam a permis d'inhiber le l'installation du phénomène de tolérance aiguë aux analgésiques morphiniques.

De plus, la **Figure 1C****,** qui reprend les résultats ci-dessus, indique que la quantité de morphine administrée en titration pour le groupe 4 est plus faible que celle qui aurait dû lui être administrée si le néfopam n'avait que des effets analgésiques **(****Figure 1B****).**

Ceci démontre donc clairement que l'effet d'inhibition de la tolérance aiguë aux morphiniques du néfopam est distinct de son effet analgésique.

## Revendications

1. Composé de formule générale (I) suivante : 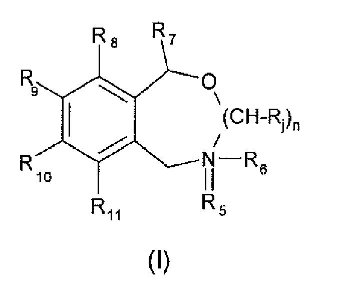 dans laquelle :
- R₅ représente O ou aucun groupement ;
- R₆ représente H ou un groupement alkyle contenant de 1 à 6 atomes de carbone ;
- n représente un nombre entier de 2 à 4 ;
- j représente un nombre entier variant de 1 à n ;
- Rⱼ, identique ou différent pour chaque carbone substitué, représente H ou un groupement alkyle contenant de 1 à 6 atomes de carbone ;
- R₇ représente un groupement phényle éventuellement substitué par un ou plusieurs groupements, identiques ou différents, choisi parmi la liste comprenant H, un groupement alkyle contenant de 1 à 6 atomes de carbone, un groupement alkoxy contenant de 1 à 6 atomes de carbones, un groupement trifluorométhyle, ou un atome d'halogène ;
- R₈, R₉, R₁₀, R₁₁ identiques ou différents représentent H, un groupement alkyle contenant de 1 à 6 atomes de carbone, un groupement alkoxy contenant de 1 à 6 atomes de carbone, un groupement trifluorométhyle, ou un atome d'halogène;
ou sel pharmaceutiquement acceptable de celui-ci,
pour son utilisation dans la prévention ou le traitement de la tolérance aiguë aux morphiniques, chez un individu ayant reçu une administration d'un analgésique morphinique.

2. Composé ou sel acceptable de celui-ci selon la revendication 1, correspondant :
- au néfopam de formule (II) suivante, 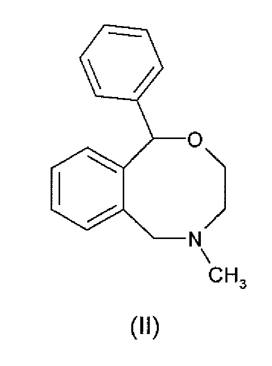
- ou au desméthyle néfopam de formule (III) suivante, 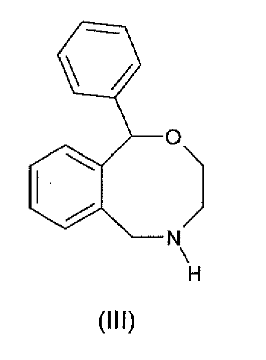
- ou au *N*-oxyde néfopam de formule (IV) suivante,

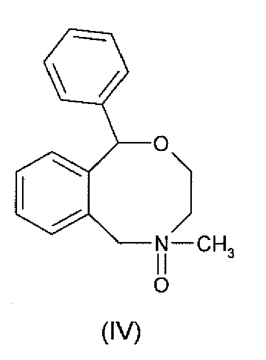

3. Composé ou sel acceptable de celui-ci selon la revendication 1 ou 2, correspondant au néfopam de formule (II) suivante,

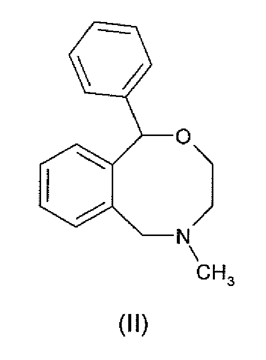

4. Composé ou sel acceptable de celui-ci selon l'une des revendications 1 à 3, pour son utilisation comme médicament convenant pour l'administration d'une dose unitaire de 1 mg à 120 mg de chlorhydrate de néfopam, pour une posologie de 1 mg/j à 120 mg/j.

5. Composé ou sel acceptable de celui-ci selon l'une des revendications 1 à 4, pour son utilisation comme médicament convenant à une administration par voie intraveineuse, par voie intramusculaire, ou par voie orale.

6. Composé ou sel acceptable de celui-ci selon l'une des revendications 1 à 5, dans lequel l'analgésique morphinique a été administré au cours d'une intervention chirurgicale.

7. Composé ou sel acceptable de celui-ci selon l'une des revendications 1 à 6, dans lequel l'analgésique morphinique est choisi parmi le groupe comprenant le fentanyl, le rémifentanil, le sufentanil, ou l'alfentanil.

8. Produits contenant :
- au moins un composé de formule (I) selon la revendication 1, et
- au moins un analgésique morphinique,
comme produit de combinaison pour une utilisation simultanée, séparée ou étalée dans le temps pour induire une analgésie chez un patient et pour prévenir ou traiter la tolérance aiguë due aux morphiniques, liée à l'utilisation dudit analgésique morphinique chez ledit patient.

9. Produits selon la revendication 8, dans lesquels le composé de formule (1) est le néfopam, le desméthyle néfopam, ou le *N*-oxyde néfopam, notamment le néfopam.

10. Produits selon la revendication 8 ou 9, dans lesquels l'analgésique morphinique est choisi parmi le groupe comprenant le fentanyl, le rémifentanil, le sufentanil, ou l'alfentanil.

11. Produits selon l'une des revendications 8 à 10, dans lesquels l'analgésique morphinique est administré au patient pour produire un effet analgésique au cours d'une intervention chirurgicale, et le composé de formule (I), notamment le néfopam, est également administré au patient durant l'intervention chirurgicale, en particulier avant le réveil dudit patient.
